(19) Europäisches Patentamt
European Patent Office
Office européen des brevets

(11) Veröffentlichungsnummer: **0 330 816 B1**

(12) **EUROPÄISCHE PATENTSCHRIFT**

(45) Veröffentlichungstag der Patentschrift: **29.11.95**

(51) Int. Cl.⁶: **A61B 19/00**, A61B 17/225, G10K 11/28

(21) Anmeldenummer: **89100526.6**

(22) Anmeldetag: **13.01.89**

Die Akte enthält technische Angaben, die nach dem Eingang der Anmeldung eingereicht wurden und die nicht in dieser Patentschrift enthalten sind.

(54) **Vorrichtung zur Erzeugung von das Wachstum pathologischen Gewebes oder dergleichen einschränkenden bzw. unterbindenden bzw. rückbildenden Ultraschallsignalformen für eine Ultraschallsendeanordnung.**

(30) Priorität: **01.03.88 DE 3806532**

(43) Veröffentlichungstag der Anmeldung:
**06.09.89 Patentblatt 89/36**

(45) Bekanntmachung des Hinweises auf die Patenterteilung:
**29.11.95 Patentblatt 95/48**

(84) Benannte Vertragsstaaten:
**FR GB IT**

(56) Entgegenhaltungen:
**EP-A- 0 282 727**
**FR-A- 2 571 635**
**GB-A- 2 140 693**
**GB-A- 2 187 840**

**C.R.Hill "Ultrasonic exposure thresholds for changes in cells and tissues" in: The Journal of the Acoustical Society of America, Vol.52, No.2 (Part 2), 1972, p.667-672**

(73) Patentinhaber: **Richard Wolf GmbH**
**Pforzheimer Strasse 32**
**D-75438 Knittlingen (DE)**

(72) Erfinder: **Riedlinger, Rainer, Dr.-Ing.**
**Bernhard Strasse 7**
**D-7500 Karlsruhe 1 (DE)**

(74) Vertreter: **Wilcken, Hugo, Dr. et al**
**Patentanwälte**
**Wilcken & Vollmann**
**Musterbahn 1**
**D-23552 Lübeck (DE)**

## Beschreibung

Die Erfindung geht aus von einer Vorrichtung gemäß dem Oberbegriff des Patentanspruches 1.

Zur medizinischen Behandlung von wucherndem Gewebe sind einerseits Geräte und operative Verfahren der Chirurgie, andererseits gerätelose, nichtoperative Methoden der Chemotherapie sowie nichtoperative Verfahren und Geräte zur Strahlentherapie und Hyperthermie bekannt.

Operative akustische Verfahren hingegen sind in der klinischen Anwendung nur wenig verbreitet. Bei diesen Verfahren wird beispielsweise ein mechanisch schwingender Metall-Hohlstift als Behandlungswerkzeug verwendet. Ein solches Gerät ist beispielsweise in der DE-A-35 27 586 beschrieben. Ähnliche Geräte zur ultraschall-operativen Gewebsentfernung sind aus den US-A-35 26 219 und 35 89 363 bekannt. Der erzielbare Störungsgrad der akustisch-mechanischen Kontaktverfahren reicht von der reversiblen Vermischung des Zellinhaltes bis zur hochgradigen Desintegration von Membranen, Mitochondrien und Zellkernen.

Daneben sind viele hyperthermisch mit Ultraschall arbeitende Geräte und Verfahren bekannt, beispielsweise aus der DE-A-33 31 510, der DE-A-33 00 121 und der DE-A-31 50 513. Dessen ungeachtet müssen diese nichtoperativen akustischen Verfahren und Geräte zur Behandlung von Geweben, speziell zur Behandlung von wuchernden Geweben in Mensch und Tier, bis zum heutigen Zeitpunkt als Versuche im Stande des Experimentierstadiums angesehen werden.

Das gleiche trifft zu auf die nichtoperativen akustischen, nichthyperthermisch arbeitenden Einrichtungen zur Behandlung von wuchernden Geweben im menschlichen Körper. Die Wirkung einer Behandlung mit einem hyperthermisch arbeitenden Gerät beruht darauf, daß bei einer länger anhaltenden Temperatur von ca. 42,5° Tumore absterben. Die lokale Anhebung der Temperatur im Körperinneren bereitet dabei aber erhebliche Schwierigkeiten, da das Gewebe eine Durchblutungskühlung erfährt und somit zur wesentlichen Temperaturerhöhung relativ langanhaltende, hohe Schallintensitäten erforderlich sind. Diese können ihrerseits aber im vorgelagerten gesunden Gewebe Kavitationen und dadurch bewirkte Schädigungen verursachen.

Auf dem Gebiet der nichtoperativen akustischen und nichthyperthermisch wirkenden Wucher-Gewebebehandlungs-Verfahren sind nur wenige Hinweise veröffentlicht worden. So ist beispielsweise aus der DE-A-35 44 344 eine Vorrichtung zur Hemmung des Wachstums von pathologischen Neubildungen im lebenden Organismus bekannt, bei der durch fokussierende Stoßwellen Blutgefässe thromboisiert werden. Hierzu wird in guter Näherung ein einmaliger Überdruckimpuls erzeugt.

Aus der Veröffentlichung "Prog. appl. Microcirc.", vol. 12, Seiten 41 bis 50, 1987, sind die folgenden Wirkungen von Stoßwellen auf Gewebe bekannt: Vaskokonstriktion, Stasis in Kapillaren, Mikrohämmorhagie, Austritt von Makromolekülen aus Venülen, Entstehung von Konglomeraten in Venülen, Blutungen und Hämatombildungen, Zerstörung von Gefäßwandungen, Zerstörung endotheler Zellen und Austritt roter Blutzellen aus Gefäßen. Hinweise auf eine Tumorbehandlung sind dieser Veröffentlichung nicht zu entnehmen.

Auf der 64. Tagung der Vereinigung der Bayerischen Chirurgen im Juli 1987 in Bad Reichenhall berichteten Wilmer, Delius und Brendel über den Effekt von Stoßwellen auf Tumorzellen in vitro anhand der sogenannten Angehrate. Auf derselben Veranstaltung referierten Goeth, Königsberger, Conzen und Brendel über die Auswirkung von Stoßwellen auf die Tumormikrozirkulation und das Tumorwachstum bei syrischen Goldhamstern. Es wurde von den folgenden Effekten berichtet: Mikrohämmorhagie, Extravasation von Makromolekülen, intravasale Thromboisierungen und temporäre Arteriolen-Konstriktion. Bei einer repetiven Behandlung mit Stoßwellen-Serien wurden Tumoreinschmelzungen erzielt.

In dem Aufsatz von C. R. Hill, "Ultrasonic Exposure Threshold for Changes in Cells and Tissues" in J. Acoust. Soc. Am., Vol. 52, No. 2 (Part 2), 1972, pp. 667-672, wird über Schwellwerte von Ultraschallanwendungen in Zellen und Geweben zwecks Feststellung von Veränderungen darin berichtet. In seinem ersten Teil wird ausführlich darüber berichtet, wann in Flüssigkeiten mit niedriger Viskosität Kavitation durch Ultraschall eintritt. In seinem zweiten Teil sind, soweit Gewebeversuche vorgenommen worden sind, Feststellungen enthalten, daß bei halb- oder subharmonischen Ultraschallsignalen keine Kavitationstätigkeit in Brustgeweben nachgewiesen werden konnte, obwohl bei Anwendung der gleichen Schallparameter in einem weiten Bereich von Flüssigkeit eine Kavitationsaktivität festgestellt werden konnte. Lediglich am Rande ist unter dem Aspekt nichtkavitationaler Mechanismen in allgemeiner Form die Anwendung in der Tumortherapie erwähnt.

In der FR-A-25 71 635 ist ein Ultraschallerzeuger für die Anwendung bei der Nierensteinzertrümmerung beschrieben, bei dem die negative Halbwelle des erzeugten Ultraschallsignales mittels zweier paralleler Folienteile unterdrückt wird. Betriebsparameter für den Ultraschallerzeuger sind nicht angegeben. Für die Bekämpfung von krankem Gewebe ohne Schädigung des umgebenden gesunden Gewebes sind aus dieser Vorrichtung keinerlei Schlüsse zu ziehen.

Vor dem aufgezeigten Stand der Technik ist es nun die Aufgabe der vorliegenden Erfindung, eine Vorrichtung für die nichtoperative akustische und nichthyperthermisch wirkende Behandlung von ausgewählten Gewebebereichen anzugeben, welche Pulsschallsignalformen erzeugt, die zwar das Wachstum von pathologischem Gewebe dauerhaft einschränken, unterbinden bzw. rückbilden, jedoch umgebendes gesundes Gewebe weder zeitweilig noch dauerhaft schädigen sollen.

Diese Aufgabe wird gelöst durch die Vorrichtung mit den kennzeichnenden Merkmalen des Patentanspruches 1.

Weitere vorteilhafte Ausbildungen der Vorrichtung ergeben sich aus den Unteransprüchen.

Die von der Vorrichtung im Fokus der von ihr angesteuerten Pulsschallsendeanordnung generierten Pulsschallsignalformen erzeugen in dem zu behandelnden Gewebe infolge lokaler Schalldruckkonzentration eine kontrollierte Kavitation mit druckphasenunterstütztem Kollaps, wobei keine Temperaturerhöhung mit hyperthermischer Wirkung erzeugt wird. Die extrem kurzen Schallpulse beinhalten dabei so wenig vom Gewebe absorbierbare Energie, daß im durchbluteten lebenden Gewebe praktisch keine beschallungsbedingte Temperaturerhöhung nachgewiesen werden kann.

Um die absorbierbare akustische Leistung zu verringern, sind zwischen den Beschallungspulsen längere Pausen vorgesehen, deren Dauer ein Vielfaches der Signaldauer betragen und diese auch um mehrere Größenordnungen übersteigen kann. Als typisch können beispielsweise eine Signaldauer von 20 $\mu$s und eine Pausendauer von 0,5 s gelten. Dabei ermöglicht die lange Pausendauer eine weitgehende oder vollständige Rückbildung gasgefüllter Kavitationsbläschen und somit wiederum dem nachfolgenden kurzzeitigen Beschallungspuls die Erzeugung definierter kontrollierter Kavitation.

Hierbei und nachfolgend wird unter definierter Kavitation verstanden, daß der Zeitpunkt des Auftretens von Kavitation exakt steuerbar ist und der Ort eng im Fokusgebiet umgrenzt bleibt. Kontrollierte Kavitation hingegen bedeutet hierbei und nachfolgend, daß der Radius der Kavitationsbläschen präzise auf kleine Werte einstellbar ist.

Die erfindungsgemäße Vorrichtung hat den Vorteil, daß großes und unkontrolliertes Blasenwachstum, wie bei länger andauernder intensiver Ultraschallbeschallung hervorgerufen, vermieden wird. Ein weiterer Vorteil gegenüber Stoßwellen erzeugenden Einrichtungen ist darin zu sehen, daß die bei Stoßwellen extrem hohen Schalldruckamplituden vermieden werden.

Des weiteren ist bei der Wirkung des Kurzzeitschallpulses vorteilhaft, daß die in der (den) Unterdruckphase(n) des Schallpulses erzeugten winzigen Kavitationsblasen nicht nur vom Umgebungsdruck und der Blasenoberflächenspannung unterstützt kollabieren, sondern daß die Überdruckphase(n) des Kurzzeit-Schallsignales den Blasenkollaps unterstützen. Sind die erzeugten winzigen Kavitationsblasen gasfrei oder zumindest gasarm, so werden durch deren Kollapse viele winzige lokale Stoßwellenquellen geschaffen, deren Stoßintensitäten erheblich größer sind als beim freien (also nicht druckphasenunterstützten) Blasenkollaps.

Sowohl das Auftreten definierter kontrollierter Kavitation als auch die vorstehend beschriebene Erzeugung vieler winziger Schallstöße im räumlich auf das zu behandelnde Gewebe begrenzten Fokusgebiet tragen zur Lösung des aufgabengemäß angestrebten Zieles bei.

Zwar macht die Vielfalt der zu behandelnden Gewebe eine Anpassung der schalltechnischen Parameter des Schallpulssignales erforderlich, es können jedoch durchaus die Parametergrenzen quantitativ angegeben werden. Da Kurzzeitschallpulse, wie bekannt, ein kontinuierliches, je nach Signalform ein welliges, aber jedenfalls kein reines Linienspektrum besitzen, wird hier die Frequenzmittellage des Maximums im Frequenzgehalt der Kurzzeitschallpulse als "Hauptfrequenz" bezeichnet und als Parameter genannt. Die Parametergrenzen sind:

Hauptfrequenz f des Schallpulssignales:

$$20 kHz < f < 10 MHz$$

Dauer T des Schallpulssignales:

$$100 ns < T < 100 \mu s$$

Amplitude p- der Unterdruckphase(n):

$$2 \times 10^5 Pa < |p-| < 2 \times 10^8 Pa$$

Amplitude p + der Druckphase(n):

$$0 < |p+| < 4 \times 10^8 Pa$$

Wiederholungsrate r (Pulsfolgefrequenz):

$$60 s^{-1} < r < 1/(5T)$$

Darüber hinaus sind weitere Parameter für die Erzeugung von Schallpulsen knapp unterhalb der Kavitationsschwelle bzw. für die Erzeugung kontrollierter Gewebskavitationen folgende:

Hüllkurvengestalt des Schallpulssignales, Beginn des Schallsignales mit einer Überdruck- oder Zugphase.

Zur Behandlung eines bestimmten Gewebetypes, welcher an einer vorgegebenen Körperpartie lokalisiert ist, erfolgt die Wahl der Parameter innerhalb der angegebenen Grenzen nach klinischer Erfahrung. Es kommt dabei auf die Absicht an, ob aus der akustisch erzeugten mechanischen Bewegung der Bestandteile des wuchernden Zellgebietes zum einen mechanisch verursachte, gravierende Veränderungen der Gewebsumgebung und/oder zum anderen auch Veränderungen der Gewebszellen selbst bewirkt werden sollen.

Diese Veränderungen manifestieren sich zum einen in einer Verminderung, Hemmung oder gar Unterbrechung der Blutzufuhr, sowie der akustisch induzierten Störung der bisherigen Ernährungsweise der Zelle und/oder zum anderen in einer Zerstörung der Zellbausteine (Risse der Zellmembranen, Zerstörung von Bausteinen des Zellinhaltes), nicht aber in einer Zerstörung des strukturellen Zusammenhaltes des Zellenverbandes.

Grundsätzlich sind die Hauptfrequenz, die Dauer des Schallpulssignales und die Amplitude und Dauer des Unterdrucksignales die wesentlichen Größen, welche die Kavitationsschwelle in einem vorgegebenen Gewebe bestimmen. Je höher die Hauptfrequenz, je kürzer die Schallpulsdauer und die Dauer des Unterdrucksignales und je niedriger dessen Amplitude ist, desto unwahrscheinlicher ist das Auftreten von Kavitation.

Als besonders vorteilhaft hat sich eine Hauptfrequenz zwischen 0,2 und 4 MHz erwiesen. Damit ist die Dauer einer einzelnen Negativphase der applizierten Schallwellen im Fokus kürzer als 2,5 $\mu$s.

Die Hüllkurvengestalt des Schallpulssignales steuert den zeitlichen Verlauf des durch gleichgerichtete Diffusion entstehenden Blasenwachstums. Als besonders vorteilhaft haben sich Hüllkurven mit zeitlich abfallendem Verlauf erwiesen, insbesondere exponentielle (Fig. 4), rampenförmige (Fig. 6) und treppenartige (Fig. 9) Hüllkurven, wobei je nach Behandlungsproblem die geeignete Hüllkurve auszuwählen ist.

Grundsätzlich kann die verwendete Schallsendeanordnung als Verbund zwischen fokussierenden Reflektoren mit piezoelektrischen Mosaikstrahlern mit teilabsorbierendem Backing oder im Verbund mit magnetischen Burst-Schallsendern oder im Verbund mit Schallquellen nach dem Wirbelstrom- ,Paralleldraht- oder Magnetostriktions-Prinzip ausgebildet sein.

Die Schallsendeanordnung kann aber auch mit einer an sich bekannten, nach dem Dampfblasen-Explosions-Prinzip arbeitende Schallquelle ausgeführt sein. Hierfür ist es von Vorteil, diese mit zwei fokussierenden Reflektoren auszurüsten, wovon der eine schallweich, der andere schallhart reflektiert und welche bezüglich der Schallquelle räumlich so versetzt sind, daß im gemeinsamen externen Fokusgebiet des ersten Reflektors und des aus dem zweiten Reflektor mit dem ersten Reflektor gebildeten Doppelreflektors ein Schallsignal alternierenden Vorzeichens erzeugt wird.

Gemäß einer weiteren vorteilhaften Ausbildung der zusammen mit der eingangs genannten Vorrichtung zum Einsatz kommenden Schallsendeanordnung mit einem Schallsender, der nach dem Dampfblasen-Expansionsprinzip arbeitet, weist diese zwei fokussierende, einander gegenüberstehende Reflektoren auf, wovon der eine schallweich, der andere schallhart reflektiert, wobei die Reflektoren bezüglich der Schallquelle räumlich so angeordnet und versetzt sind, daß im gemeinsamen externen Fokusgebiet der Reflektoren ein Pulsschallsignal alternierenden Vorzeichens erzeugt wird. Auch eine koaxiale, ellipsoid-ringförmige Ausgestaltung der Reflektoren ist möglich.

Die Erfindung ist nachstehend anhand der anliegenden Zeichnungen näher erläutert. Es zeigen:

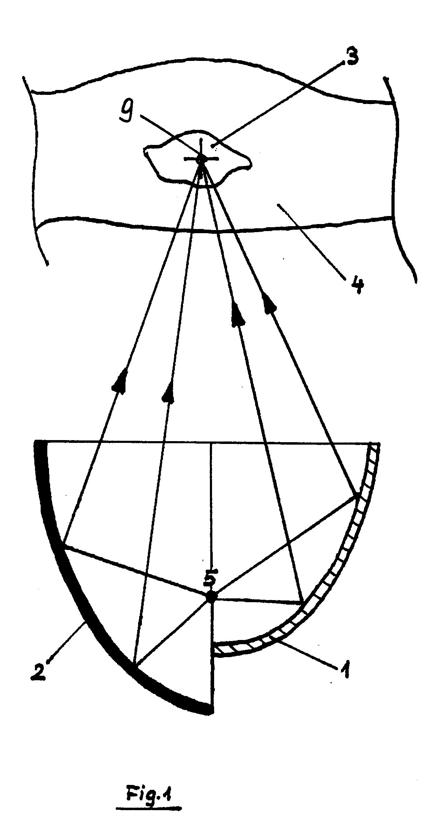

Fig. 1      eine Vorrichtung nach der Erfindung mit zwei je verschiedene Raumsektoren erfassenden Reflektoren und einer Kurzzeit-Pulsschallquelle,

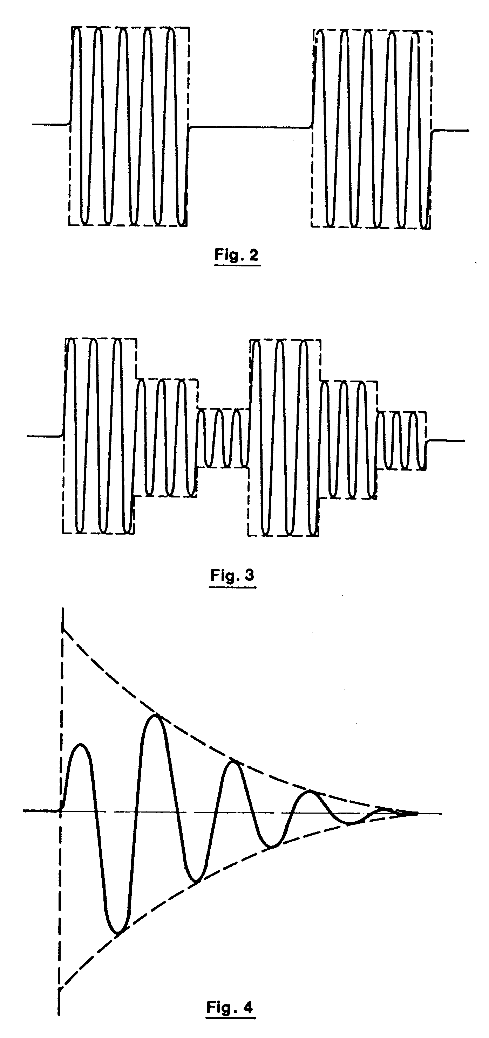

Fign. 2 bis 6      Schallpulssignale mit unterschiedlichen Hüllkurven,

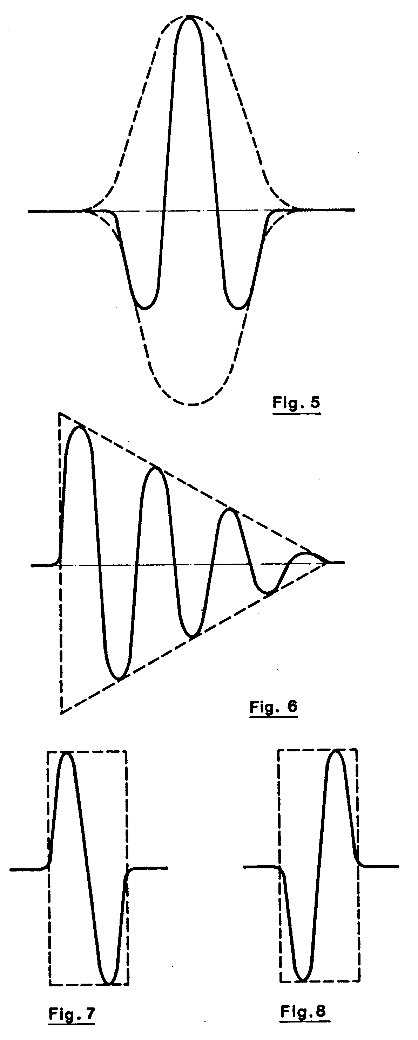

Fig. 7      einen Schallpuls mit einer Periode, wobei der Puls mit einer Überdruckphase beginnt,

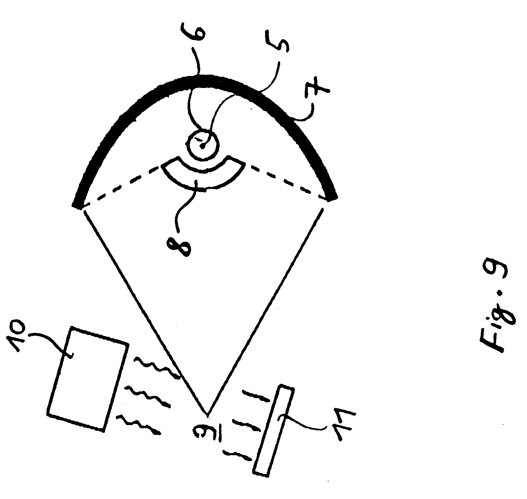

Fig. 8      einen Schallpuls mit einer Periode, wobei der Puls mit einer Unterdruckphase beginnt,

Fig. 9      einen mittels eines ersten Doppelreflektors fokussierenden Burstschallsender, der bei Verwendung einer Explosionsschallquelle als Pulsschallsender zum Einsatz kommt,

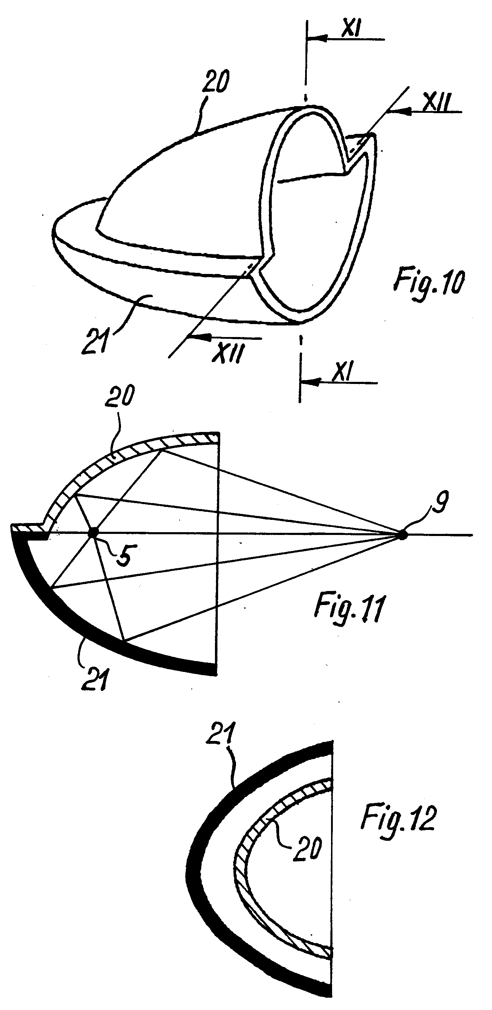

Fig. 10      einen zweiten Doppelreflektor zur Anwendung in Kombination mit einem Burstschallsender in perspektivischer Ansicht,

Fig. 11      der Doppelreflektor gemäß Fig. 10 im vertikalen Schnitt,

Fig. 12      der Doppelreflektor gemäß Fig. 10 im horizontalen Schnitt.

Figur 1 zeigt eine erfindungsgemäße Vorrichtung mit einer Pulsschallsendeanordnung 1, 2, 5, welche die weiter unten angegebenen Signale in einem externen Fokusgebiet 9 erzeugt. Die Schallsendeanordnung besteht aus einem Schallsender 5, dessen abgestrahlter Schall über Reflektoren 1, 2 in den menschlichen Körper 4 eingeleitet und dort in dem zu behandelnden Gewebe 3 gebündelt wird.

Erfindungswesentlich ist, mit welchen Pulsschallsignalformen das zu behandelnde Gewebe zum Zweck des Einschränkens bzw. vollständigen Unterbindens von dessen Wachstum bzw. dessen Rückbildung beaufschlagt wird. Hier zeigen die Fign. 2 bis 6 geeignete Signalformen, die von der erfindungsgemäßen Vorrichtung abgegeben, d. h. im Fokusgebiet jenes Fokus erzeugt werden können, der außerhalb der Reflektoren liegt und in den das zu behandelnde Gewebe positioniert wird.

Es kommt für das Entstehen einer das Wachstum des Gewebes beeinträchtigenden Wirkung zunächst nicht darauf an, ob die im Gewebe erzeugte kontrollierte Kavitation durch eine periodische Rechteck-Schallpulsfolge gemäß Fig. 2 oder durch eine Pulsfolge mit einem treppenförmigen Hüllkurvenverlauf gemäß Fig. 3 erzeugt wird. Da aber die primär entstehenden Radien der Kavitationsbläschen sowie deren Wachstum durch die Hüllkurvenamplitude und deren Zeitverlauf bestimmt werden, ist - wie eingangs erwähnt - eine dem Behandlungsproblem angepaßte Hüllkurve zu wählen.

Insbesondere bieten Schallquellen nach dem Paralleldrahtprinzip hierzu geeignete Betriebsweisen. Aber auch mit anderen Quellen, wie z. B. Wirbelstromquellen, lassen sich durch pulsierende Stromzufuhr in Verbindung mit erfindungsgemäßen Quellen-Reflektor-Anordnungen Signalverläufe herstellen, deren prinzipielle Verläufe in den Fign. 2 bis 8 dargestellt sind.

Als vorteilhafte Hüllkurvengestalt hat sich eine abklingende Exponentialfunktion gemäß Fig. 4 erwiesen. Ebenso vorteilhaft als Hüllkurven haben sich die Gauß-Funktion sowie die lineare Rampenfunktion erwiesen, wie sie in den Fign. 5 und 6 dargestellt sind. Bei Verwendung derselben Hauptfrequenz und gleichen Spitzenamplituden und Dauern der Hüllkurven erzeugt die Schallimpulsfolge mit rechteckförmiger Hüllkurve das größte Blasenwachstum.

Den wichtigen Fall eines Schallimpulses mit nur einer einzigen Schwingungsperiode zeigen die Fign. 7 und 8.

Diese Pulsart ist dann von besonderem Vorteil, wenn das bipolare Schallsignal mit der Zugphase beginnt, vgl. Fig. 8. Dadurch kann bei geeigneter Wahl der Periodendauer und der Zugamplitude ein einzelner schalldruckunterstützter Kavitationsblasenkollaps erzielt werden, wobei dieser Kollaps sekundäre Stoßwellen lokal erzeugt. Deren Stoßamplituden übersteigen erheblich die jeweiligen Stoßamplituden, welche von normal kollabierenden Kavitationsblasen hervorgerufen werden, wenn diese also nicht schalldruckphasenunterstützt, sondern nur durch den Umgebungsdruck und durch die Blasenoberflächenspannung veranlaßt kollabieren.

Besteht der Schallpuls aus einer Einzelschwingung (Fig. 7 und 8), so kann das Wachstum von Kavitationsblasen durch gleichgerichtete Diffusion vermieden werden. Dazu ist allerdings Voraussetzung, daß die Pausen zwischen den Schallpulsen um mindestens etwa eine Größenordnung länger sind als die Schallpulsdauer.

Eine zunehmende Wiederholungsrate erhöht die Wahrscheinlichkeit für das Auftreten von neuen Kavitationsbläschen und vergrößert durch den Effekt der gleichgerichteten Diffusion den Blasenradius, was letztendlich zum Zerreißen der Zellmembran und damit zur Zerstörung des pathologischen Gewebes führt. Wird eine die Zellbestandteile unmittelbar angreifende Beschallung mit der Wirkung einer mechanischen Zellschädigung angestrebt, so ist eine hohe Wiederholungsrate zu wählen. Niedrige Wiederholungsraten sind dagegen für die angestrebte Störung der Ernährungssituation der Zellen des zu behandelnden pathologischen Gewebes günstiger, da durch die Schaffung eines blutflußhemmenden Gewebeareales eine Abkoppelung des beschallten Gewebebereiches von der Blutzufuhr bewirkt wird.

Daß die Schallsendeanordnung verschiedenartige Expansionsschallquellen enthalten kann, ist bereits erwähnt worden. Anhand der Fign. 9 bis 12 ist nun eine weitere Ausführung beschrieben, die bei Verwendung einer Explosionsschallquelle als Pulsschallsender in Verbindung mit der erfindungsgemäßen Vorrichtung zum Einsatz kommen kann.

Wird demnach als Schallquelle eine Explosionsschallquelle verwendet, so bedarf es für die Erzeugung der vorstehend beschriebenen Signalformen einer speziellen Ausbildung der Sendeanordnung. In Fig. 9 ist eine derartige Anordnung dargestellt. Bei dieser Sendeanordnung wird ein Raumsektor der von der Explosionsschallquelle 5 erzeugten Überdruckwelle 6 an einem harten (alternativ weichen) Ellipsoid-Reflektor 7, ein anderer Raumsektor der Stoßwelle zunächst an einem weichen (alternativ harten) Hohlkugelkalotten-Reflektor 8 und nachfolgend am Reflektor 7 reflektiert, so daß im externen Fokusgebiet 9 ein Druckpuls von einem Zugpuls (alternativ ein Zugpuls von einem Druckpuls) gefolgt wird. Eine besonders vorteilhafte Lösung ist dann gegeben, wenn der Reflektor 8 gerade den vom Reflektor 7 nicht erfaßten Raumsektor erfaßt, und zwar so, wie dies in Fig. 9 dargestellt ist. Dabei wird nämlich einerseits die unfokussierte Schalleinstrahlung in den Körper gerade unterbunden und andererseits praktisch die gesamte, von der Explosionsschallquelle erzeugte Überdruckwelle gebündelt. Als Expansions- bzw. Explosionsschallquellen kommen beispielsweise Wirbelstromquellen und elektrohydraulische Quellen in Betracht. Bei letzteren kann durch hohe momentane Energiezufuhr in die durch Funkenüberschlag erzeugte Plasmablase die Expansion schneller als die Schallgeschwindigkeit des die Blase umgebenden Mediums gemacht werden, so daß man diese Quelle auch als Explosionsquelle bezeichnet. Sie erzeugt dann statt eines Überdruckpulses (mit relativ langsamem Druckanstieg) eine Stoßwelle mit einem nahezu als Druck-Sprung zu bezeichnenden, extrem kurzen Druckanstieg.

Ist der Reflektor 8 hart, der Reflektor 7 hingegen weich, so wird im externen Fokusgebiet 9 ein Doppelpuls erzeugt, dessen Einzelpulse mit einer Zugphase beginnen.

Durch die Wahl der Reflektorgeometrien lassen sich die erfaßten Raumsektoren einstellen, womit die Möglichkeit geboten wird, die Druck- und Zugamplituden des im externen Fokusgebiet 9 entstehenden Schallbursts nach Bedarf unterschiedlich einzustellen.

Es kann aber auch eine Fokussiereinrichtung gemäß der Fign. 10, 11 und 12 Verwendung finden. Diese Einrichtung besteht aus zwei Teilellipsoiden 20, 21, deren Fokusgebiete zwar koinzident sind, deren Reflektoren aber unterschiedliche Schallaufzeiten von der im internen Fokus befindlichen Schallquelle zum im externen Fokus befindlichen Tumorgewebe verursachen, wobei ein Reflektor hart, der andere weich reflektiert.

Die letztgenannte Anordnung vermeidet gegenüber der erstgenannten das Problem einer möglichen Kavitationsentstehung im Quellenfokus der Fig. 9, welches insbesondere bei Verwendung eines weichen Reflektors 8 besteht.

Die erfindungsgemäße Vorrichtung umfaßt vorzugsweise neben dem Kurzzeit-Schallpulssender eine Ortungseinrichtung, die in den Figuren nicht dargestellt ist. Die Ortungseinrichtung ermöglicht das Detektieren von Wuchergeweben im menschlichen Körper in ans ich bekannter Weise durch Ultraschall-A- oder Ultraschall-B-Scanning oder durch ultraschalltomographische Methoden.

Insbesondere kann dabei der Behandlungs-Schallsender einem Ultraschall-A-Scanner zugeordnet oder mit diesem sogar identisch sein. Das A-Scanning erlaubt dabei den Nachweis der kontrolliert erzeugten Kavitation und insbesondere auch den unmittelbar nach der Beschallung registrierbaren Schwund von Kavitationsnebeln im Gewebe.

Die Ortungseinrichtung kann auch in bekannter Weise aus einem Röntgensystem, MR-System oder aus einem PET-System bestehen.

In Fig. 9 ist beispielsweise ein Ortungssender 10 und ein Ortungsempfänger 11 angedeutet, welche die Beobachtung des Fokus- und Behandlungsgebietes 9 ermöglichen.

Eine übliche Positioniereinrichtung erlaubt es, das zu behandelnde Wuchergewebe in das externe Fokusgebiet 9 des Pulsschall-Senders zu bringen. Dabei wird entweder der Schallsender oder der Patient mit einem in den drei Raumrichtungen verfahrbaren Verschiebesystem bewegt. Das Verschiebesystem ist dabei wahlweise translatorisch und/oder rotatorisch beweglich.

**Patentansprüche**

1. Vorrichtung zur Erzeugung von die Wachstumstendenz pathologischen Gewebes (3) in Lebewesen vermindernden Ultraschallsignalformen mittels einer fokussierte Schallwellen aussendenden Ultraschallsendeanordnung (5, 7, 8), wobei die Schallwellen dieser Anordnung mit Hilfe eines Koppelmediums in das zu behandelnde Gewebe (3) übertragen werden, dadurch gekennzeichnet,

   - daß die Ultraschallsendeanordnung eine Pulsschallquelle (5) enthält und mit zwei fokussierenden Reflektoren (7, 8; 20, 21) ausgerüstet ist, wovon der eine schallweich, der andere schallhart reflektiert und die bezüglich der Pulsschallquelle (5) räumlich so zueinander versetzt sind, daß im gemeinsamen externen Fokusgebiet (9) des ersten Reflektors (7) und des aus dem zweiten Reflektor (8) mit dem ersten Reflektor (7) gebildeten Doppelreflektors (7, 8) ein Pulsschallsignal erzeugt wird,

   - und daß die Pulsschallquelle (5) zur Erzielung einer kontrollierten Kavitation in dem externen Fokusgebiet (9) in dem zu behandelnden Gewebe (3) ein Pulsschallsignal erzeugt, dessen kurzzeitig abgestrahlte Schallpulse in dem Gewebe mindestens eine Zugphase mit negativer Schalldruckamplitude, deren Betrag größer als $2 \times 10^5$ Pa ist, aufweisen, dessen Hauptfrequenz über 20 kHz liegt, dessen durch eine Hüllkurve bestimmte Schallimpulsdauer T weniger als 100 μs beträgt und dessen Wiederholungsrate kleiner als 1/(5T) ist.

2. Vorrichtung nach Anspruch 1, dadurch gekennzeichnet, daß die Pulsschallquelle (5) eine nach dem Dampfblasen-Explosionsprinzip arbeitende Schallquelle ist.

3. Vorrichtung nach Anspruch 1, dadurch gekennzeichnet, daß die Pulsschallquelle (5) eine nach dem Wirbelstrom-Prinzip arbeitende Schallquelle ist.

4. Vorrichtung nach Anspruch 1, dadurch gekennzeichnet, daß die Pulsschallquelle (5) eine nach dem Paralleldraht-Prinzip arbeitende Schallquelle ist.

5. Vorrichtung nach Anspruch 1, dadurch gekennzeichnet, daß die Pulsschallquelle (5) eine nach dem Magnetostriktions-Prinzip arbeitende Schallquelle ist.

6. Vorrichtung nach Anspruch 1, dadurch gekennzeichnet, daß die Pulsschallquelle (5) ein nach dem piezoelektrischen Prinzip arbeitende Schallquelle mit teilabsorbierendem Backing ist.

7. Vorrichtung nach einem der Ansprüche 1 bis 6, dadurch gekennzeichnet, daß die Reflektoren (20, 21) der Ultraschallsendeanordnung aus zwei sich gegenüberstehenden Teilellipsoiden mit koizidenten internen Fokusgebieten, aber unterschiedlichen Schallaufzeiten bis zum externen Fokusgebiet (9) bestehen, wobei sich die Schallquelle (5) im ersten Fokus befindet.

8. Vorrichtung nach wenigstens einem der Ansprüche 1 bis 7, dadurch gekennzeichnet, daß der zeitliche Verlauf der Hüllkurve des Pulsschallsignales im externen Fokus (9) eine Exponentialfunktion darstellt.

9. Vorrichtung nach wenigstens einem der Ansprüche 1 bis 7, dadurch gekennzeichnet, daß der zeitliche Verlauf der Hüllkurve des Schallimpulssignales im externen Fokus (9) eine Rampenfunktion darstellt.

10. Vorrichtung nach wenigstens einem der Ansprüche 1 bis 9, dadurch gekennzeichnet, daß das Pulsschallsignal mit einer Zugphase beginnt.

11. Vorrichtung nach wenigstens einem der Ansprüche 1 bis 10, dadurch gekennzeichnet, daß der wesentliche spektrale Hauptfrequengehalt zwischen 0,2 und 4 MHz liegt und damit die Dauer einer einzelnen Zugbelastungshalbwelle des applizierten Pulsschallsignales im externen Fokus (9) kürzer als 2,5 $\mu$s ist.

**Claims**

1. A device for the production of ultrasonic signal forms reducing the growth tendency of pathological tissue (3) in living creatures by means of an ultrasonic transmission arrangement (5, 7, 8) emitting focussed sound waves, in which the sound waves of this arrangement are transferred by means of a coupling medium into the tissue (3) which is to be treated, characterised in that

 - the ultrasonic transmission arrangement contains a pulse sound source (5) and is equipped with two focussing reflectors (7, 8; 20, 21), one of which reflects in a sound-absorbent manner and the other of which reflects in a non-absorbing manner with respect to sound, and which are staggered three-dimensionally with respect to each other in relation to the pulse sound source (5) so that in the common external focus field (9) of the first reflector (7) and of the double reflector (7, 8) formed from the second reflector (8) with the first reflector (7), a pulse sound signal is produced,

 - and that the pulse sound source (5), to achieve a controlled cavitation in the external focus field (9) in the tissue (3) which is to De treated, produces a pulse sound signal, the momentarily radiated sound pulses of which have in the tissue at least a draught phase with negative sound pressure amplitude, the amount of which is greater than 2 x 10 Pa, the main frequency of which lies above 20 kHz, the sound impulse duration T of which, determined by an envelope curve, amounts to less than 100 $\mu$s and the repetition rate of which is less than 1/- (5T).

2. A device according to Claim 1, characterised in that the pulse sound source (5) is a sound source operating according to the steam bubble explosion principle.

3. A device according to Claim 1, characterised in that the pulse sound source (5) is a sound source operating according to the eddy current principle.

4. A device according to Claim 1, characterised in that the pulse sound source (5) is a sound source operating according to the parallel wire principle.

5. A device according to Claim 1, characterised in that the pulse sound source (5) is a sound source operating according to the magnetostriction principle.

6. A device according to Claim 1, characterised in that the pulse sound source (5) is a sound source operating according to the piezoelectric principle with partially absorbing backing.

7. A device according to one of Claims 1 to 6, characterised in that the reflectors (20, 21) of the ultrasonic transmission arrangement consist of two partial ellipsoids, standing opposite each other, with coincident internal focus fields, out with different sound travel times up to the external focus field (9), in which the sound source (5) is situated in the first focus.

8. A device according to at least one of Claims 1 to 7, characterised in that the time characteristic of the envelope curve of the pulse sound signal in the external focus (9) represents an exponential function.

9. A device according to at least one of Claims 1 to 7, characterised in that the time characteristic of the envelope curve of the sound impulse signal in the external focus (9) represents a ramp function.

10. A device according to at least one of Claims 1 to 9, characterised in that the pulse sound signal begins with a draught phase.

11. A device according to at least one of Claims 1 to 10, characterised in that the essential spectral main frequency content lies between 0.2 and 4 MHz and hence the duration of an individual tensile load half wave of the applied pulse sound signal in the external focus (9) is shorter than 2.5 $\mu$s .

**Revendications**

1. Dispositif destiné à produire des formes de signaux ultrasonores réduisant la tendance à la croissance de tissus pathologiques (3) dans des organismes vivants, au moyen d'un agencement d'émission d'ultrasons (5, 7, 8) émettant des ondes sonores focalisées, les ondes sonores de cet agencement étant transmises aux tissus (3) à traiter, à l'aide d'un milieu de couplage, caractérisé

    - en ce que l'agencement d'émission d'ultrasons contient une source d'impulsions sonores (5) et est équipé de deux réflecteurs (7, 8; 20, 21) de focalisation, dont l'un produit une réflexion acoustique diffuse et l'autre une réflection acoustique directe, et qui, relativement à la source d'impulsions sonores (5), sont décalés l'un par rapport à l'autre dans l'espace, de sorte que dans la région focale externe (9), commune, du premier réflecteur (7) et du réflecteur double (7, 8) formé par le second réflecteur (8) avec le premier réflecteur (7), est produit un signal d'impulsions sonores,

    - et en ce que la source d'impulsions sonores (5), en vue de l'obtention d'une cavitation contrôlée dans la région focale externe (9), dans les tissus (3) à traiter, produit un signal d'impulsions sonores, dont les impulsions sonores, rayonnées pendant une faible durée, présentent, dans les tissus, au moins une phase de traction à amplitude de pression acoustique négative, d'une valeur supérieure à $2 \times 10^5$ Pa, dont la fréquence principale se situe au-delà de 20 kHz, dont la durée d'impulsion sonore T, définie par une courbe enveloppe, est inférieure à 100 $\mu$s, et dont le taux de répétition est inférieur à 1/(5T).

2. Dispositif selon la revendication 1, caractérisé en ce que la source d'impulsions sonores (5) est une source sonore travaillant selon le principe de l'explosion de bulles de vapeur.

3. Dispositif selon la revendication 1, caractérisé en ce que la source d'impulsions sonores (5) est une source sonore travaillant selon le principe des courants tourbillonnaires.

4. Dispositif selon la revendication 1, caractérisé en ce que la source d'impulsions sonores (5) est une source sonore travaillant selon le principe de fils métalliques en parallèle.

5. Dispositif selon la revendication 1, caractérisé en ce que la source d'impulsions sonores (5) est une source sonore travaillant selon le principe de la magnétostriction.

6. Dispositif selon la revendication 1, caractérisé en ce que la source d'impulsions sonores (5) est une source sonore travaillant selon le principe piézoélectrique, et comportant une base partiellement absorbante.

7. Dispositif selon l'une des revendications 1 à 6, caractérisé en ce que les réflecteurs (20, 21) de l'agencement d'émission d'ultrasons sont constitués par deux ellipsoïdes partiels en regard l'un de l'autre, dont les régions focales internes coïncident, mais dont les durées de propagation du son jusqu'à la région focale externe (9) sont différentes, la source sonore (5) se trouvant dans le premier foyer.

8. Dispositif selon l'une au moins des revendications 1 à 7, caractérisé en ce que l'allure en fonction du temps de la courbe enveloppe du signal d'impulsions sonores dans le foyer externe (9), représente une fonction exponentielle.

9. Dispositif selon l'une au moins des revendications 1 à 7, caractérisé en ce que l'allure en fonction du temps de la courbe enveloppe du signal d'impulsions sonores dans le foyer externe (9), représente une fonction de déclivité linéaire.

**10.** Dispositif selon l'une au moins des revendications 1 à 9, caractérisé en ce que le signal d'impulsions sonores débute avec une phase de traction.

**11.** Dispositif selon l'une au moins des revendications 1 à 10, caractérisé en ce que la teneur spectrale essentielle en fréquence principale se situe entre 0,2 et 4 Mhz, et la durée d'une demi-onde de contrainte de traction individuelle du signal d'impulsion sonore appliqué dans le foyer externe (9), est ainsi plus courte que 2,5 $\mu$s.

Fig.1

**Fig. 2**

**Fig. 3**

**Fig. 4**

Fig. 5

Fig. 6

Fig. 7

Fig. 8

Fig. 9

Fig.10

Fig.11

Fig.12